# EUROPEAN PATENT SPECIFICATION

(11) **EP 3 297 612 B1**
(45) Date of publication and mention of the grant of the patent: **24.03.2021**
(21) Application number: 16728613.7
(22) Date of filing: 19.05.2016
(51) Int. Cl.: A61K 9/48, A61K 31/138

(54) **PHARMACEUTICAL COMPOSITION COMPRISING ATOMOXETINE AND METHOD FOR THE PREPARATION THEREOF**
PHARMAZEUTISCHE ZUSAMMENSETZUNG ENTHALTEND ATOMOXETIN UND METHODE ZU DEREN HERSTELLUNG
COMPOSITION PHARMACEUTIQUE COMPRENANT ATOMOXETINE ET SON PROCÉDÉ DE PRÉPARATION

(30) Priority: 22.05.2015 GR 20150100240
(43) Date of publication of application: 28.03.2018
(73) Proprietor: Pharmathen S.A., 15351 Pallini Attikis (GR)
(72) Inventor: KARAVAS, Evangelos, 153 51 Attikis (GR); KOUTRIS, Efthymios, 153 51 Attikis (GR); SAMARA, Vasiliki, 153 51 Attikis (GR); KOUTRI, Ioanna, 153 51 Attikis (GR); KALASKANI, Anastasia, 153 51 Attikis (GR); KAKOURIS, Andreas, 153 51 Attikis (GR); GOTZAMANIS, George, 153 51 Attikis (GR)
(86) International application number: PCT/EP2016/000831
(87) International publication number: WO 2016/188623

(56) References cited:
- EP-A1- 1 379 492
- WO-A1-2007/031801
- KR-A- 20150 002 234
- ANONYMOUS: "Strattera (Atomoxetine HCl)", INTERNET CITATION, 10 January 2006 (2006-01-10), pages 1-25, XP002743713, Retrieved from the Internet: URL:http://www.fda.gov/ohrms/dockets/docke ts/06p0209/06P-0209-EC3-Attach-1.pdf [retrieved on 2015-08-27]

## Description

### TECHNICAL FIELD OF THE INVENTION

The present invention relates to a stable pharmaceutical formulation for oral administration containing a therapeutically effective quantity of Atomoxetine or salt or polymorph thereof and a method for the preparation.

### BACKGROUND OF THE INVENTION

Atomoxetine (brand name: Strattera) is a norepinephrine reuptake inhibitor approved for the treatment of attention-deficit hyperactivity disorder (ADHD). Classified as a norepinephrine (noradrenaline) reuptake inhibitor, atomoxetine is approved for use in children, adolescents, and adults. However, its efficacy has not been studied in children under six years old. Its primary advantage over the standard stimulant treatments for ADHD is that it has no abuse potential. The initial therapeutic effects of atomoxetine usually take 2-4 weeks to become apparent. A further 2-4 weeks may be required for the full therapeutic effects to be seen. Its efficacy may be less than that of stimulant medications. Unlike α2 adrenoceptor agonists such as guanfacine and clonidine, atomoxetine's use can be abruptly stopped without significant withdrawal effects being seen.

Atomoxetine is not a psychostimulant and is not an amphetamine derivative. In a randomized, double-blind, placebo-controlled, abuse-potential study in adults comparing effects of atomoxetine and placebo, atomoxetine was not associated with a pattern of response that suggested stimulant or euphoriant properties.

The pharmacokinetics of atomoxetine in children and adolescents are similar to those in adults. Atomoxetine is rapidly and almost completely absorbed after oral administration, reaching mean maximal observed plasma concentration (Cmax) approximately 1 to 2 hours after dosing. The absolute bioavailability of atomoxetine following oral administration ranged from 63% to 94%, depending upon inter-individual differences in the modest first-pass metabolism. Atomoxetine can be administered with or without food. Atomoxetine is widely distributed and is extensively (98%) bound to plasma proteins, primarily albumin.

In adults, the presence of symptoms of ADHD that were pre-existing in childhood should be confirmed. Third-party corroboration is desirable and STRATTERA should not be initiated when the verification of childhood ADHD symptoms is uncertain. Diagnosis cannot be made solely on the presence of one or more symptoms of ADHD. Based on clinical judgment, patients should have ADHD of at least moderate severity as indicated by at least moderate functional impairment in 2 or more settings (for example, social, academic, and/or occupational functioning), affecting several aspects of an individual's life.

Suicide-related behavior (suicide attempts and suicidal ideation) has been reported in patients treated with atomoxetine. In double-blind clinical trials, suicide-related behaviors were uncommon, but more frequently observed among children and adolescents treated with atomoxetine compared to those treated with placebo, where there were no events. In adult double-blind clinical trials there was no difference in frequency of suicide related behavior between atomoxetine and placebo. Patients who are being treated for ADHD should be carefully monitored for the appearance or worsening of suicide related behavior.

Atomoxetine belongs to antidepressant therapeutic category and is a white or almost white powder. It is freely soluble in water, soluble in anhydrous ethanol and practically insoluble in heptane. The Atomoxetine HC1 salt is designated chemically as (3,R)-N-Methyl-3-(2-methylphenoxy)-3-phenylpropan-1-amine hydrochloride.

It's been administered in the form of a hard gelatin capsule of varying size, appearance, fill weight and drug loading starting from 10mg up to 100mg single dose.

EP 1 379 492 B1 discloses Atomoxetine formulations in the form of hard capsules comprising starch and a silicone oil lubricant.

Other attempts have been reported like in EP 1 715 856 B1 & EP 1 758 557 B1 for alternative pharmaceutical formulations of Atomoxetine having different release profile, different mechanism of drug release and manufacturing process.

Although each of the patents above represent suitable pharmaceutical compositions for administering Atomoxetine, there still exists a need for alternative stable pharmaceutical compositions.

### SUMMARY OF THE INVENTION

It is, therefore, an object of the present invention to provide an alternative stable solid dosage formulation for oral administration containing atomoxetine or pharmaceutical acceptable salt or polymorph thereof, as an active ingredient.

A major object of the present invention is to provide an oral solid dosage formulation comprising Atomoxetine, which can be formulated into dosage forms of different strengths by proportionally adjusting the amounts of the pharmaceutically acceptable excipients, as well as the pharmaceutical active compound. The dosage forms of the present invention are characterized by pharmacotechnical linearity, without having any effect on the dissolution profile and bioavailability of said drug.

A further approach of the present invention is to provide a method for the preparation of such dosage forms for oral administration containing atomoxetine that is easy to manufacture, fast and economic.

In accordance with the above objects of the present invention, an immediate release pharmaceutical composition for oral administration is provided comprising Atomoxetine or a pharmaceutically acceptable salt or polymorph thereof as an active ingredient and an effective amount of a pharmaceutically acceptable suspending agent.

Other objects and advantages of the present invention will become apparent to those skilled in the art in view of the following detailed description.

### DETAILED DESCRIPTION OF THE INVENTION

For the purposes of the present invention, a pharmaceutical composition comprising a pharmaceutical active ingredient is considered to be "stable" if said ingredient degrades less or more slowly than it does on its own and/or in known pharmaceutical compositions.

It is well known that during the development of any pharmaceutical dosage form the impurities should be kept at very low limits, as said impurities might be toxic and harmful for the humans. Moreover, the impurities diminish the potency of pharmaceutical compositions during storage. They can also act as catalysts or intermediates in chemical reactions and change the drug into another form. Therefore, there is a need to develop a formulation that is stable and does not promote the degradation of the active pharmaceutical ingredient.

A very important parameter for the pharmaceutical industry is the achievement of linearity between the strength and the dosage form weight. The present invention provides solid dosage forms that exhibit linearity between the strength of the drug formulation and the total mass of the formulation, by proportional increase of the amounts of the drug substance and the excipients in the formulation. Such an approach provides the ability to perform a bioequivalence study to only one strength and extrapolate the results to other strengths of the dosage form, thus providing a far more economic solution.

Another embodiment of the present invention is the use of a cost effective process for the preparation of solid dosage forms. Hard gelatin capsules were chosen as the pharmaceutical dosage form in the present invention. Hard gelatin capsules are widely used because a) swallowing is very easy, b) the shells have no taste and the drugs which are not having pleasant taste and smell can be administered, c) they can be manufactured in different colors and d) the drug will be released easily as there is no compaction. They are not generally used for the compounds that are highly soluble because the drug will be delivered all at once. When it comes to a decision at the end of phase II in a production line, which dosage form will be developed for the market, high production costs of hard gelatin capsule products are generally assumed. This assumption is valid if the production costs are limited to the comparison of the excipient costs only. When taking into account the total manufacturing costs, which include the hidden costs coming from process equipment, GMP space required, total production time, in-process controls, analytical, cleaning and validation work the comparison easily turns out in favor of the capsule formulation.

In the present invention motivated by the market tendency to provide dosage forms comprising natural excipients, it was decided to use hard gelatin capsules that are SLS free in contrast to those used for the product that is already in the market. Unexpectedly, it was found that the capsule shell does have an impact on the dissolution profile of the specific dosage form.

The marketed product uses a high amount of pregelatinized starch. Said excipient is known for its swelling properties. This property in connection with the amount used entraps the active substance in the gel layer that is formed thus causes a delay to the release. For the marketed product this is been solved by the capsule shell type. In the present invention the use of SLS free capsules, in order to achieve the desired profile, made the use of a suspending agent necessary.

Various suspending agents where tested such as lactose, sodium starch glycolate, croscarmellose sodium, sodium lauryl sulfate and various types of silicon dioxide.

Colloidal silicone dioxide (Aerosil) has a well-established use in pharmaceutical industry as a glidants in compositions. However, in the present invention it was proved to function as a suspending agent that helps the pharmaceutically active ingredient not to get entrapped within the gel formed by the pregelatinized starch. It was added in an amount of less than 5% w/w, preferably of 4% w/w or less & more preferably of 2% w/w or less.

Similarly functional for the present invention was found to be the material Syloid® 244 FP. It is a synthetic amorphous silica appearing as a white free flowing powder. It meets the test requirements for silicon dioxide as described in the latest edition of United States Pharmacopoeia/National Formulary (USP/NF), for silica, dental type as described in the latest edition of the European Pharmacopoeia (EP), and for the hydrated silicon dioxide as described in the latest edition of Japanese Pharmaceutical Excipients. It is a high pore volume silica gel with a large internal surface area. It has a strong affinity for moisture and organic based liquids. Syloid® 244 FP silica can adsorb up to 1.6 ml of liquid per gram & is particularly recommended as a glidant, tabletting-aid and carrier for pharmaceutical and personal care products.

Moreover, the pharmaceutical compositions of the present invention may also contain one or more additional formulation excipient such as diluents, disintegrants, binders, lubricants, glidants and flavoring agents, provided that they are compatible with the active ingredient of the composition, so that it does not interfere with it in the composition and in order to increase the stability of the drug and the self-life of the pharmaceutical product.

Diluents may be, for example, microcrystalline cellulose, dextrates, dextrose, fructose, mannitol, sorbitol, sucrose, xylitol, maltose, maltodextrin, maltitol.

Disintegrants may be selected from alginic acid, carbon dioxide, guar gum, methylcellulose, polacrilin potassium, poloxamer, sodium alginate.

Binders may be, for example, alginic acid, carbomer, ethyl cellulose, gelatin, liquid glucose, guar gum, hydroxyethyl cellulose, methylcellulose, polydextrose, polyethylene oxide.

Also, at least a lubricant is incorporated into the formulation Lubricants may be, for example, talc, magnesium stearate, calcium stearate, glyceryl behenate, hydrogenated castor oil, stearic acid, dimethicone.

Glidants are used to promote powder flow by reducing interparticle friction and cohesion. These are used in combination with lubricants as they have no ability to reduce die wall friction. Glidants, may be, for example, dimethicone, calcium silicate, calcium phosphate tribasic.

Flavouring agents are used to mask potential unpleasant tasting active ingredients and improve the likelihood that the patient will complete a course of medication. Such agents are particularly useful in case of orodispersible, chewable and sublingual tablets. Flavouring agents may be, for example, mint powder, menthol, vanillin, aspartame, acesulfame potassium, saccharin.

According to the present invention, dry granulation was used as the process for the preparation of dosage forms of Atomoxetine. It is one of the most economical methods, as only standard equipment is used and the product is usually characterized by satisfactory physical resistance with no need for special packaging.

The main advantages of said process are that less equipment and space is required. Further, said process eliminates the need for binder solution, heavy mixing equipment and the costly and time consuming drying step required for wet granulation.

The process is characterized by the following steps:
- Weighing of active ingredient and excipients;
- Sifting of all excipients and dry blending with the active ingredient;
- Blending of the above mixture with a glidants;
- Sizing of the bulk to eliminate any clumps;
- Encapsulation

The following examples illustrate preferred embodiments in accordance with the present invention.

### EXAMPLES

### Reference Example 1:

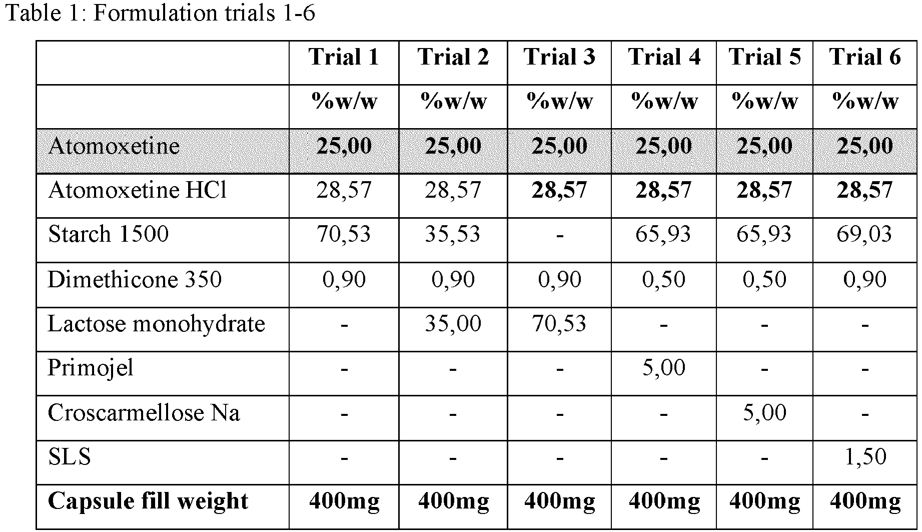

All composition trials were manufactured with the following process:
- Weighing of active ingredient and excipients
- Sifting of all excipients and dry blending with the active ingredient
- Blending of the above mixture with Dimethicone
- Sizing of the bulk to eliminate any clumps
- Encapsulation in SLS free capsules

A first composition (trial 1) was manufactured having the same excipients as the marketed product with the difference that is was encapsulated in an SLS free hard gelatin capsule.

The dissolution profile obtained (table 2) seriously deviated in the first 10-15 minutes from that of the marketed product. In order to enhance the release of the active ingredient from the dosage form a series of known dissolution promoters was incorporated in the formulation as presented in table 1 above for formulation trials 2 to 6. In trials 2 and 3 starch 1500 was replaced, in part & totally respectively, by lactose in order to avoid the swelling of starch. In trials 4 and 5 known disintegrants sodium starch glycolate and croscarmellose sodium were added to enhance dissolution. Finally, SLS was added in the composition for similar reasons. As can been seen from table 2 none of the excipients used managed to address the problem.

Even though, SLS was tested as a part of the actual composition, in contrast to the marketed product where it lies in the capsule shell, didn't have the desired effect.

**Table 2: Dissolution profiles of Strattera® & trials 1 to 6 in HCl 0.1N as dissolution medium.**

| **Time (min)** | **Strattera®** | **Trial 1** | **Trial 2** | **Trial 3** | **Trial 4** | **Trial 5** | **Trial 6** |
|---|---|---|---|---|---|---|---|
| 5 | 86,83 | 21,70 | 15,15 | 49,18 | 10,56 | 14,92 | 26,89 |
| 10 | 99,96 | 79,14 | 70,15 | 66,67 | 57,93 | 65,82 | 74,13 |
| 15 | 101,12 | 90,58 | 89,30 | 76,76 | 86,68 | 85,48 | 81,35 |
| 20 | 101,39 | 95,39 | 95,26 | 84,48 | 92,51 | 92,41 | 86,02 |
| 30 | 102,09 | 98,74 | 100,07 | 95,60 | 96,28 | 96,65 | 91,27 |
| 45 | 103,04 | 101,76 | 102,17 | 101,94 | 102,92 | 99,37 | 95,47 |

### Example 2:

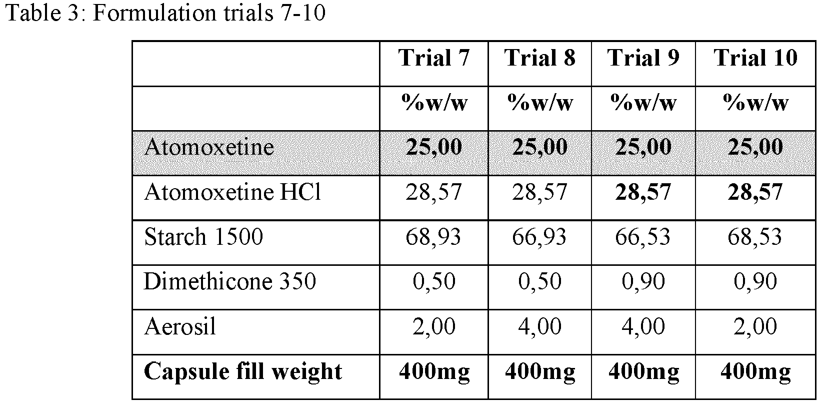

Compositions of trials 7 to 10 were all prepared according to the process described in example 1 and their dissolution profile was measured in the same conditions (table 4).

**Table 4: Dissolution profiles of trials 7 to 10 in HCl 0.1N as dissolution medium.**

| **Time (min)** | **Trial 7** | **Trial 8** | **Trial 9** | **Trial 10** |
|---|---|---|---|---|
| 5 | 51,98 | 63,51 | 46,76 | 42,68 |
| 10 | 85,35 | 96,00 | 85,61 | 82,54 |
| 15 | 94,14 | 99,74 | 96,93 | 93,80 |
| 20 | 97,56 | 100,51 | 99,52 | 97,25 |
| 30 | 99,94 | 102,09 | 101,22 | 98,81 |
| 45 | 101,86 | 102,50 | 102,35 | 100,36 |

It is evident from the results that Aerosil enhanced Atomoxetine dissolution rate by preventing the drug entrapment into the gel formed. It can be observed that there is an increase of dissolution rate when the Aerosil concentration is increased and the amount of Dimethicone is low. On the other hand when the amount of Dimethicone is on the higher concentration selected, there is no effect depicted from the increase of Aerosil. Overall, the four compositions where acceptable, however it is worth mentioning that trials 9 and 10 had better granule flowability which is a further advantage in large scale production and even more trial 10 had a slightly more compact bulk which leads to a faster and easier encapsulation.

### Example 3:

Furthermore, another silica type was investigated namely Syloid® 244FP & a weight reduction of the capsule content (table 5).

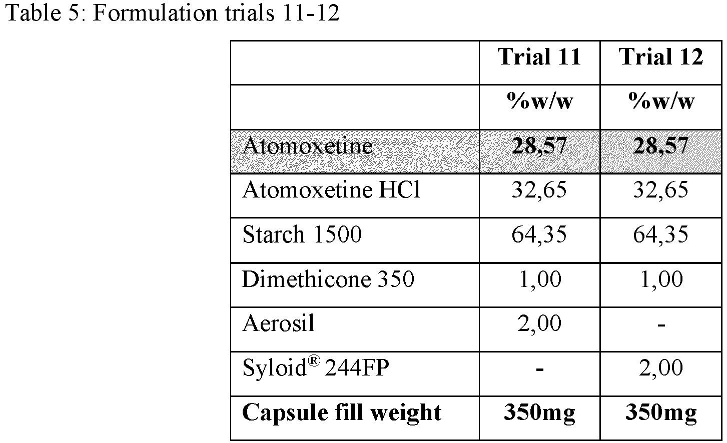

The manufacturing process was the same as in previous examples as well as the dissolution profile conditions.

**Table 6: Dissolution profiles of trials 11 & 12 in HCl 0.1N as dissolution medium.**

| **Time (min)** | **Trial 11** | **Trial 12** |
|---|---|---|
| 5 | 85,67 | 52,47 |
| 10 | 93,28 | 78,83 |
| 15 | 95,65 | 91,91 |
| 20 | 97,79 | 96,79 |
| 30 | 100,45 | 100,69 |
| 45 | 101,45 | 101,84 |

It is evident from the above that reduction of the capsule fill weight significantly improved the dissolution release of Atomoxetine and that the use of Syloid® 244FP marginally fulfills the standards of the present invention.

Formulation trials 1 and 11 were loaded into stability chambers. Stability data upon storage at zero time & 6months under long term (25oC/60% RH), intermediate (30oC/60% RH) and accelerated storage conditions (40°C/75% RH) are presented in the following table.

**Table 7: Stability results of trial 1 & 11**

| | **Formulation Trial 1** | | | | **Formulation Trial 11** | | | |
|---|---|---|---|---|---|---|---|---|
| | **Zero time** | **6 months** | | | **Zero time** | **6 months** | | |
| | | **25°C** | **30°C** | **40°C** | | **25°C** | **30°C** | **40°C** |
| Impurity B S-(+) isomer (NMT 0.5%) | 0,02 | 0,14 | 0,15 | 0,17 | 0,01 | 0,08 | 0,08 | 0,08 |
| Impurity D m-isomer (NMT 0.2%) | ND | ND | ND | ND | ND | ND | ND | ND |
| Total Unknown (NMT 0.2%) | 0,07 | 0,18 | 0,16 | 0,14 | 0,08 | 0,13 | 0,09 | 0,10 |
| **Total** (NMT 1.0%) | **0,09** | **0,32** | **0,31** | **0,31** | **0,09** | **0,21** | **0,17** | **0,18** |

What can be surprisingly seen from table 7 above is that the use of colloidal silicon dioxide provides a stabilizing effect. The results above show good stability of the product of the present invention, as well as good compatibility between the drug substance and the excipients proposed by the present invention. Moreover, the good results regarding the physicochemical characteristics, the excellent stability of the drug product, as well as the particular manufacturing process followed, indicate the advantages of the present invention.

The composition of trial 11 was prepared for active ingredient content of 10, 18, 25, 40, 60, 80 & 100mg with total weight of capsule fill weight 35, 63, 87.5, 140, 210, 280 & 350mg respectively. The dissolution profile results were excellent and directly comparable with those of the marketed product which does not follow this linear increase of active content to weight. Thus, for the composition as proposed hereby, the important aspect of linearity which pertains economic benefits for any company is achieved.

While the present invention has been described with respect to the particular embodiments, it will be apparent to those skilled in the art that various changes and modifications may be made in the invention.

## Claims

1. A pharmaceutical composition in the form of a hard gelatin capsule for oral administration comprising Atomoxetine or a pharmaceutical acceptable salt or polymorph thereof as an active ingredient and an amount of 2% to 4% w/w of silicon dioxide acting as suspending agent, wherein the hard gelatin capsule is SLS free.

2. The pharmaceutical composition according to claim 1, wherein Atomoxetine or a pharmaceutical acceptable salt, or polymorph thereof is in amount of 25% to 40% w/w.

3. The pharmaceutical composition according to claim 1, wherein the silicon dioxide is a colloidal silicon dioxide or an amorphous silicon dioxide.

4. The pharmaceutical composition according to any preceding claim, wherein it further comprises at least one pharmaceutically acceptable excipient selected from diluents, lubricants, glidants and flavouring agents.

5. The pharmaceutical composition according to any preceding claim, wherein said composition further comprises pregelatinized starch and dimethicone.

6. A process for the preparation of a pharmaceutical composition according to claim 1, which comprises the steps of:
- Weight of Atomoxetine or a pharmaceutical acceptable salt or polymorph thereof as the active ingredient, the silicon dioxide in an amount of 2% to 4% w/w and any optional pharmaceutically acceptable excipient;
- Sifting of all excipients and dry blending with the active ingredient;
- Blending of the above mixture with a lubricant;
- Sizing of the bulk to eliminate any clumps;
- Encapsulation in SLS free hard gelatin capsules.

## Patentansprüche

1. Pharmazeutische Zusammensetzung zur oralen Verabreichung in der Arzneiform einer harten Gelatinekapsel bestehend aus Atomoxetin oder aus einem pharmazeutisch verwendbaren Salz oder Polymorph davon als Wirkstoff und Siliciumdioxid in einem Gewichtsprozent von 2% bis 4% als Suspensionsmittel, wobei die harte Gelatinekapsel SLS-frei ist.

2. Pharmazeutische Zusammensetzung nach Anspruch 1, **dadurch gekennzeichnet, dass** Atomoxetin oder das pharmazeutisch verwendbare Salz oder Polymorph davon in einem Gewichtsprozent zwischen 25% bis 40% enthalten ist.

3. Pharmazeutische Zusammensetzung nach Anspruch 1, **dadurch gekennzeichnet, dass** Siliciumdioxid ein kolloidales Siliciumdioxid oder ein amorphes Siliciumdioxid ist.

4. Pharmazeutische Zusammensetzung nach einem der vorhergehenden Ansprüche, **dadurch gekennzeichnet, dass** sie mindestens einen pharmazeutisch verwendbaren Hilfsstoff enthält, z.B. Spreng-/Zerfallsmittel, Schmiermittel, Gleitmittel und/oder Aromastoffe.

5. Pharmazeutische Zusammensetzung nach einem der vorhergehenden Ansprüche, **dadurch gekennzeichnet, dass** sie auch Quellstärke und Dimeticon enthält.

6. Verfahren für die Zubereitung einer pharmazeutischen Zusammensetzung nach Anspruch 1, welches aus den nachstehenden Schritten besteht:
• Bestimmung des Gewichts von Atomoxetin oder vom pharmazeutisch verwendbaren Salz oder Polymorph davon als Wirkstoff, Berechnung des Gewichtsprozents von Siliciumdioxid von 2% bis 4% sowie von den optionalen pharmazeutisch verwendbaren Hilfsstoffen;
• Alle Hilfsstoffe sieben und mit dem Wirkstoff vermischen;
• Die obere Mischung mit einem Schmiermittel vermischen;
• Größenbestimmung des Schüttgutes zur Beseitigung eventueller Klumpen;
• Verkapselung in SLS-freie harte Gelatinekapseln.

## Revendications

1. Une composition pharmaceutique revêtant la forme d'une capsule de gélatine dure à administrer par voie orale comprenant de l'Atomoxétine ou un sel pharmaceutique acceptable ou bien un polymorphe de celui-ci, en tant qu'ingrédient actif, ainsi qu'une quantité de 2% à 4% p/p de dioxyde de silicium agissant comme agent de suspension, dans lequel la capsule de gélatine dure est exempte de LSS.

2. Conformément à la 1ère revendication, la composition pharmaceutique, dans laquelle l'Atomoxétine ou un sel pharmaceutiquement acceptable, ou un polymorphe de celui-ci, est en quantité de 25% à 40% p/p.

3. Conformément à la 1ère revendication, la composition pharmaceutique, dans laquelle le dioxyde de silicium est un dioxyde de silicium colloïdal ou un dioxyde de silicium amorphe.

4. Conformément à toute revendication précédente, la composition pharmaceutique qui comprend, en plus, au moins un excipient accepté sur le plan pharmaceutique, choisi parmi les excipients diluants, lubrifiants, glissants et agents aromatisants.

5. Conformément à toute revendication précédente, la composition pharmaceutique qui comprend en outre l'amidon prégélatinisé et de la diméthicone.

6. Un procédé de préparation d'une composition pharmaceutique, conformément à la 1ère revendication, qui comprend les étapes suivantes:
- poids d'Atomoxétine ou d'un sel pharmaceutiquement acceptable ou polymorphe de ce dernier en tant qu'ingrédient actif, ainsi qu'une quantité de 2% à 4% p/p de dioxyde de silicium et tout autre excipient facultatif pharmaceutiquement acceptable;
- Tamisage de tous les excipients et mélange à sec avec l'ingrédient actif;
- Combinaison de ce mélange avec un lubrifiant;
- Dimensionnement de la masse pour éliminer les grumeaux;
- Encapsulation en gélatine dure exempte de LSS.
